# EUROPEAN PATENT SPECIFICATION

(11) **EP 2 276 742 B1**
(45) Date of publication and mention of the grant of the patent: **22.01.2014**
(21) Application number: 09731459.5
(22) Date of filing: 07.04.2009
(51) Int. Cl.: C07D 223/16, A61K 31/55, A61P 9/04

(54) **IVABRADINE HYDROBROMIDE**
IVABRADINHYDROBROMID
BROMHYDRATE D'IVABRADINE

(30) Priority: 07.04.2008 SI 200800080
(43) Date of publication of application: 26.01.2011
(73) Proprietor: KRKA, tovarna zdravil, d.d., Novo mesto, 8501 Novo mesto (SI)
(72) Inventor: SMRKOLJ, Matej, 1411 Izlake Zagorje ob Savi (SI); GOJAK, Urska, 8000 Novo Mesto (SI); KOTAR-JORDAN, Berta, 8311 Kostanjevica na Krki (SI)
(74) Representative: UEXKÜLL & STOLBERG
(86) International application number: PCT/EP2009/054159
(87) International publication number: WO 2009/124940

(56) References cited:
- US-A- 5 296 482
- US-A1- 2005 228 177
- STAHL P H ET AL: "Handbook of Pharmaceutical salts, passage" HANDBOOK OF PHARMACEUTICAL SALTS : PROPERTIES, SELECTION, AND USE, WEINHEIM : WILEY-VCH VERLAG, DE, 1 January 2002 (2002-01-01), pages 329-333,342, XP002472779 ISBN: 978-3-906390-26-0
- KUMAR ET AL: "An overview of automated systems relevant in pharmaceutical salt screening" DRUG DISCOVERY TODAY, ELSEVIER, RAHWAY, NJ, US, vol. 12, no. 23-24, 29 November 2007 (2007-11-29), pages 1046-1053, XP022370272 ISSN: 1359-6446

## Description

### FIELD OF THE INVENTION

The present invention relates to ivabradine hydrobromide of formula (I), to processes for its preparation and to pharmaceutical compositions containing it.

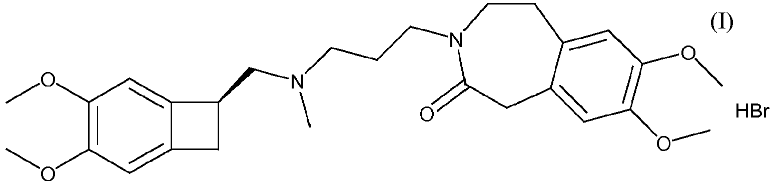

### BACKGROUND OF THE INVENTION

Ivabradine and addition salts thereof with a pharmaceutically acceptable acid have very valuable pharmacological and therapeutic properties, especially bradycardic properties, making those compounds useful in the treatment or prevention of various clinical situations of myocardial ischemia such as angina pectoris, myocardial infarct and associated rhythm disturbances, and also in various pathologies involving rhythm disturbances, especially supraventricular rhythm disturbances, and in heart failure.
The preparation and therapeutic use of ivabradine hydrochloride have been described in EP534859. New crystalline forms of ivabradine hydrochloride have been described in EP1589005, EP1695710, EP1695709, EP1707562, EP1695965, EP1775288 and EP1775287.

### BRIEF DESCRIPTION OF THE DRAWINGS

Figure 1 is an X-ray powder diffraction pattern of crystalline ivabradine hydrobromide prepared according to example 1.

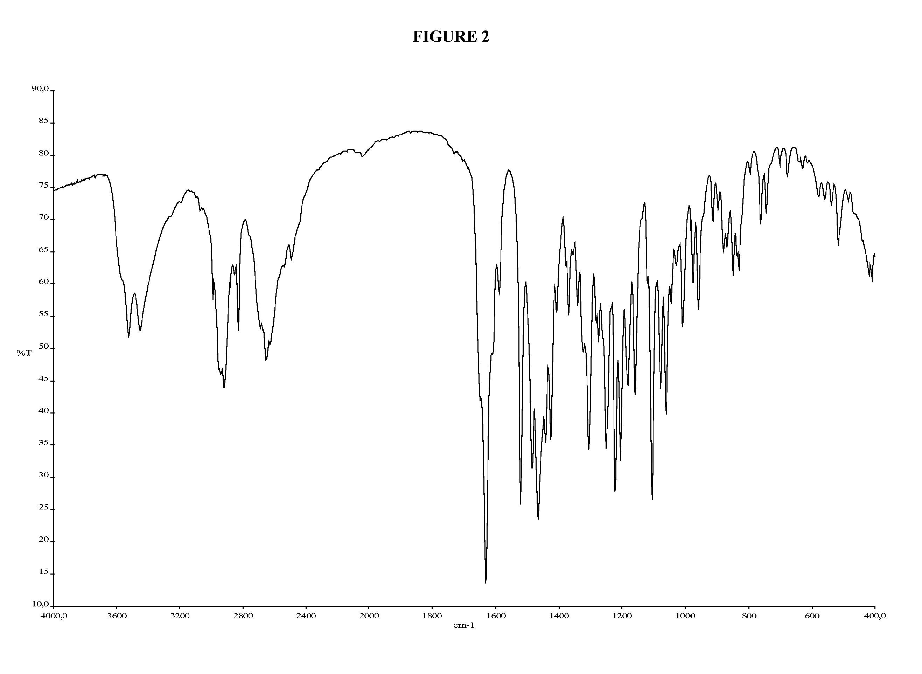

Figure 2 is a FT-IR spectrum of crystalline ivabradine hydrobromide prepared according to example 1.

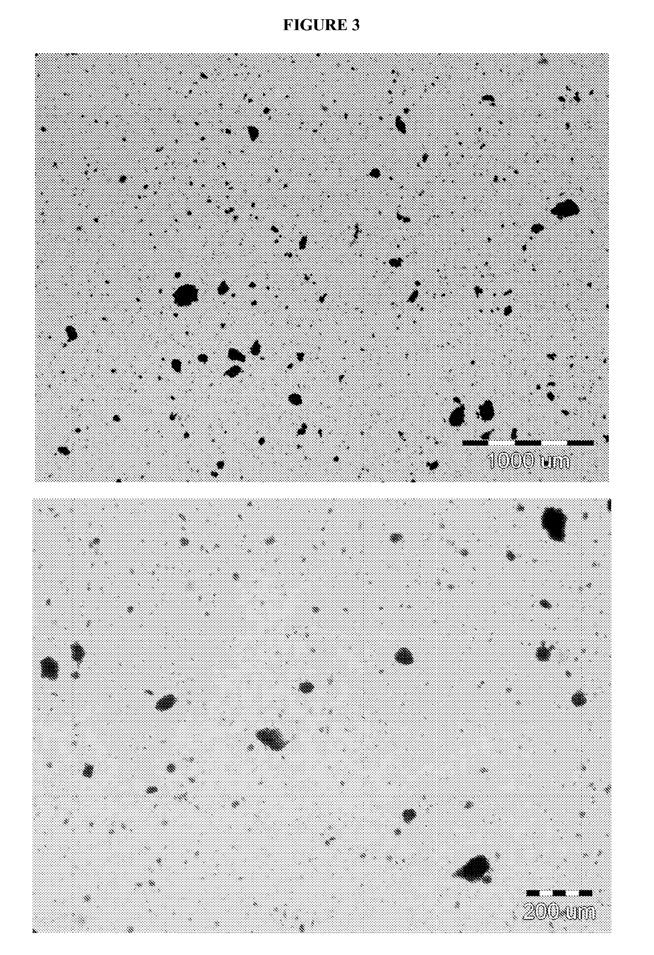

Figure 3 are pictures of particles of crystalline ivabradine hydrobromide prepared according to example 1.

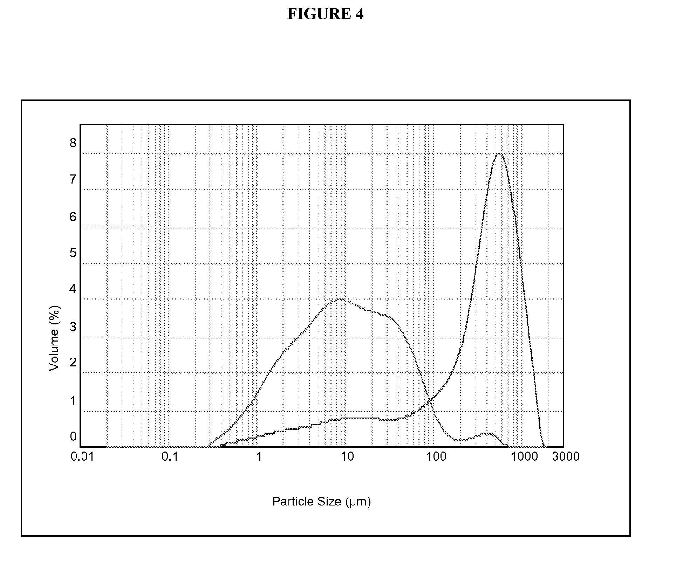

Figure 4 are particle size distribution diagrams of crystalline ivabradine hydrobromide prepared according to example 2, with and without prior two minute ultrasonication.

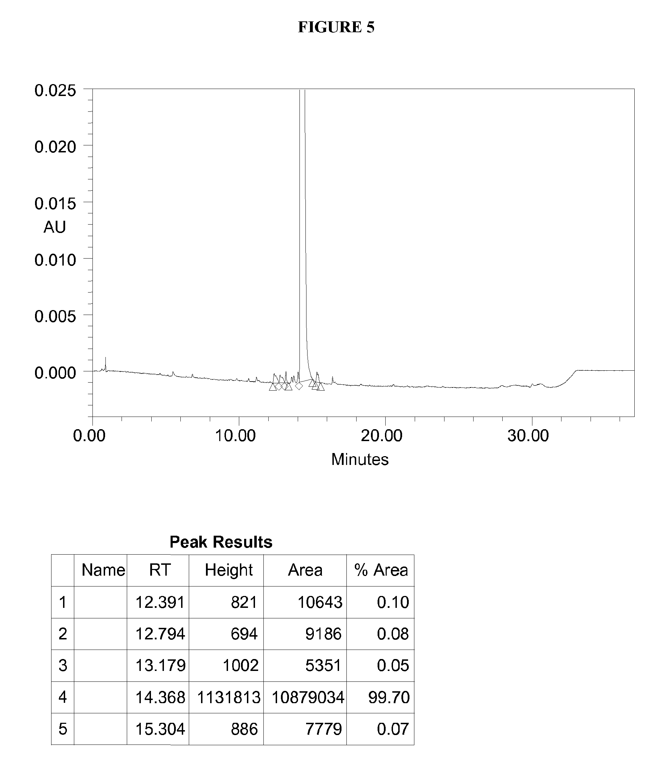

Figure 5 is UPLC chromatogram of ivabradine hydrobromide prepared according to example 1.

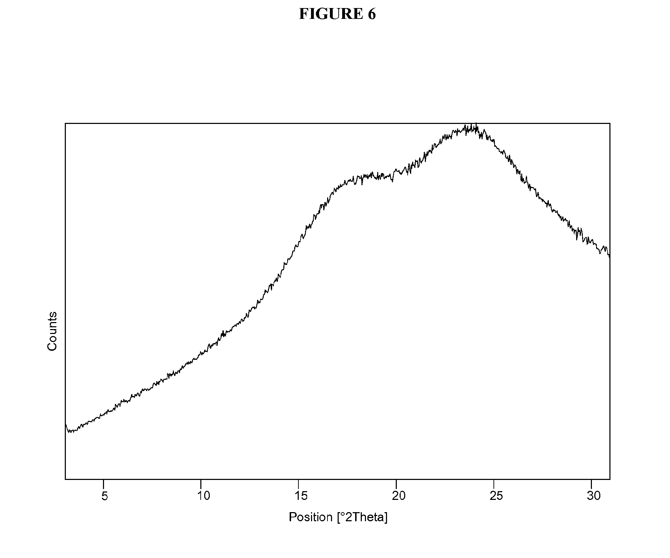

Figure 6 is an X-ray powder diffraction pattern of amorphous ivabradine hydrobromide prepared according to example 4.

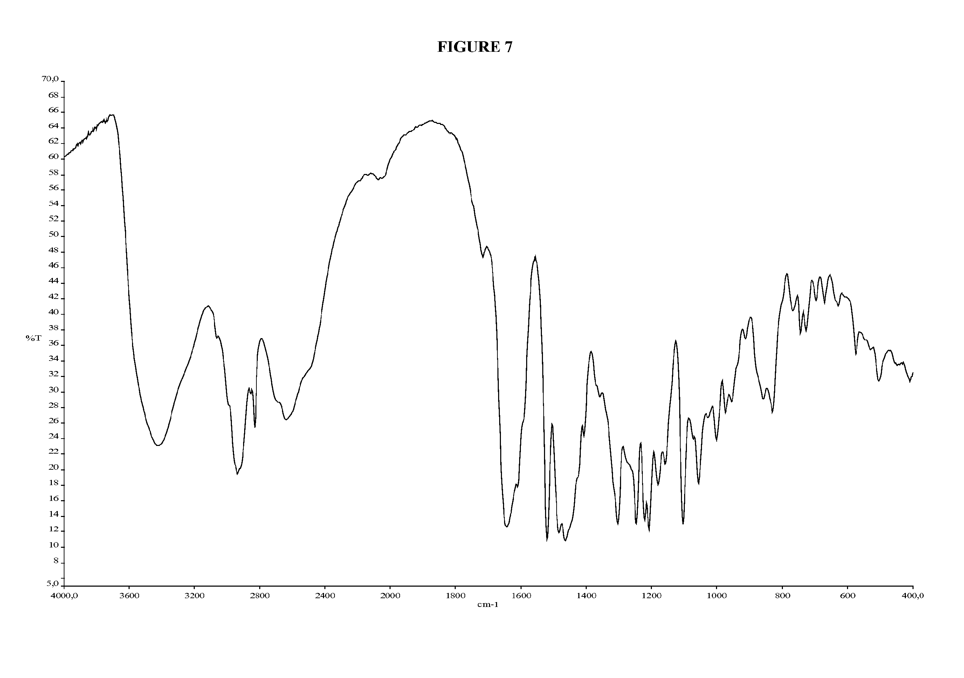

Figure 7 is a FT-IR spectrum of ivabradine hydrobromide prepared according to example 4.

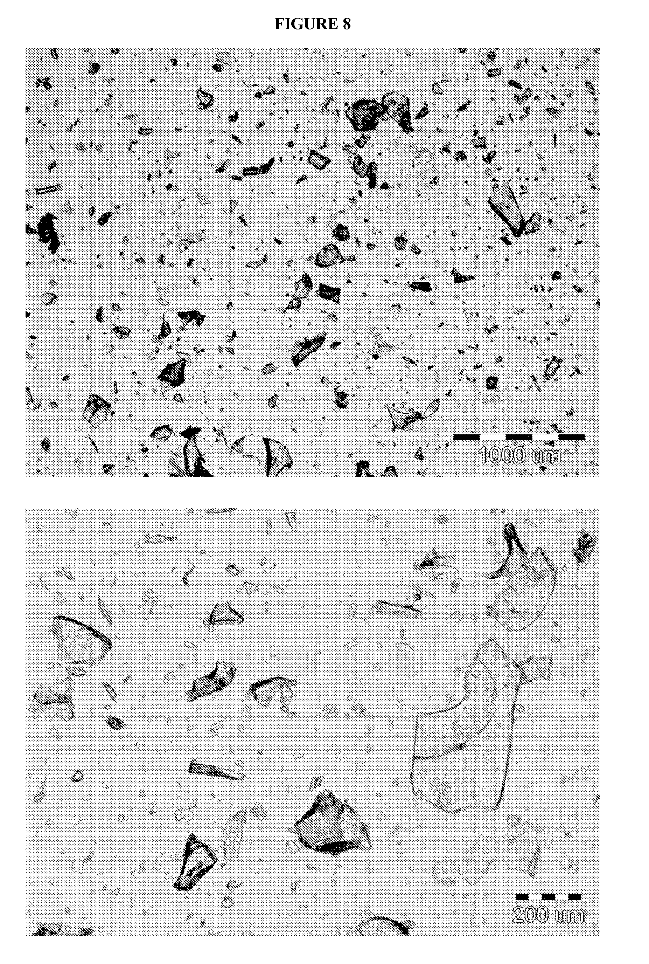

Figure 8 are pictures of particles of amorphous ivabradine hydrobromide prepared according to example 4.

The X-ray powder diffraction pattern was obtained by Philips PW3040/60 X'Pert powder diffractometer, X'celerator detector at CuKα radiation, 1.54178 Å, 3°<2θ<30°.

FT-IR spectra (KBr discs) were recorded over the wave number range of 4000-400 cm⁻¹ on Perkin Elmer FT-IR spectrometer Spectrum 100 at a resolution 4 cm⁻¹.

Images of particles were taken on a Microscope Olympus BX 50 equipped with Olympus camera DP70. Sample was prepared as suspension in paraffin oil.

The size distribution of ivabradine hydrobromide particles was determined by laser diffraction using a Malvern Mastersizer 2000 laser diffraction instrument. The samples for analysis were prepared by dispersing a weighed amount of ivabradine hydrobromide particles in isopar.

UPLC chromatogram of related substances of ivabradin was obtained on an ultra performance liquid chromatograph with UV detector. Chromatographic separation was achieved using ACQUITY UPLC BEH HSS T3, 2.1x100mm, 1.7 µm analytical column. Chromatographic conditions used were: sample concentration of 1.0mg/ml, detection at 286 nm, flow of 0.4ml/min, volume of injection 5µL. A gradient elution using mobile phase A (0.01M CH₃COONH₄ pH 7.5) and mobile phase B (CH₃CN) was employed with time table (0min 5%B, 30min 60%B, 32min 5%B, 37min 5%).

### DETAILED DESCRIPTION OF THE INVENTION

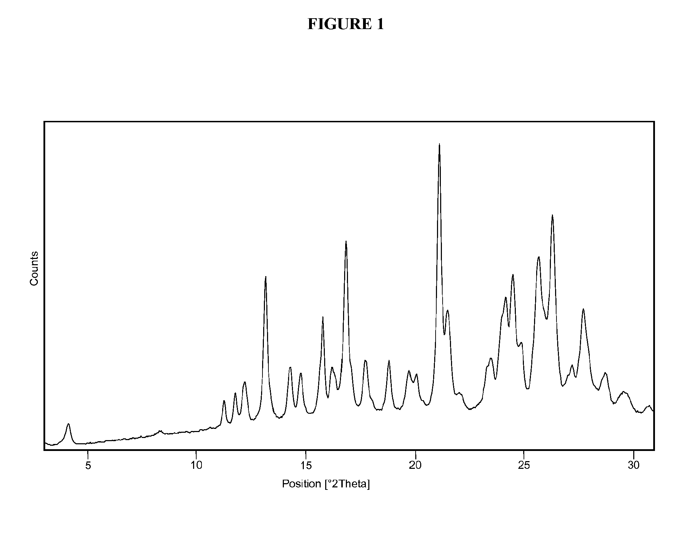

We have prepared a new salt of ivabradine which has good stability, reproducibility and in general good processability.
More specifically, the present invention relates to the ivabradine hydrobromide in a solid or dissolved state. Solid ivabradine hydrobromide can be in an amorphous or crystalline state. Crystalline ivabradine hydrobromide prepared according to present invention is characterized by an X-ray powder diffraction pattern having peaks at about 13.2, 15.8, 16.8, 21.1, 24.5 ± 0.2 degrees two-theta. Crystalline ivabradine hydrobromide can be further characterized by X-ray powder diffraction peaks at about 13.2, 15.8, 16.8, 21.1, 24.5, 25.7, 26.3, 27.7 ± 0.2 degrees two-theta. It may either be more than 90, 95%, 99%, 99.5% or 99.9% of polymorphic purity or include other polymorphic, or an amorphous form. The X-ray powder diffraction pattern of crystalline ivabradine hydrobromide is shown in Figure 1.

The new salt of ivabradine according to present invention can be prepared by common methods used in the preparation of amine salts. Crystalline ivabradine hydrobromide can be prepared by cooling solution of ivabradine hydrobromide to precipitate crystalline ivabradine hydrobromide. Crystalline or amorphous ivabradine hydrobromide can also be prepared by evaporating the solvent from ivabradine hydrobromide solution or by addition of antisolvent to ivabradine hydrobromide solution. Suitable solvents for preparation of ivabradine hydrobromide can be selected from the group consisting of water, nitriles, ketones, esters, ethers, alcohols, sulfoxides, amides, halogenated hydrocarbons or/and any mixture thereof. Preferably, the solvent is water, methanol, ethanol, acetonitrile, DMSO, acetone, ethyl methyl ketone, ethyl acetate, i-propyl acetate, methylen chloride or/and any mixture thereof.

Ivabradine solutions or suspensions can be prepared by mixing the solution of ivabradine and a solution of hydrogen bromide. Hydrogen bromide solution is preferably aqueous or alcoholic solution, most preferably aqueous solution such as 48% aqueous solution.

According to a particularly preferred embodiment of the present invention ivabradine hydrobromide is prepared by:
(i) dissolving ivabradine in a suitable solvent, preferably in methylene chloride ;
(ii) adding a solution of hydrogen bromide, preferably an alcoholic solution or more preferably an aqueous solution, most preferably a 48 % aqueous solution of hydrogen bromide;
(iii) evaporating the solvent to dryness, preferably under vacuum.

Step (i) is preferably performed at a temperature within the range of 20 to 25°C.

The process preferably comprises the following additional steps:
(iv) Redissolving or dispersing the product of step (iii) in a second solvent, preferably in ethyl acetate;
(v) maintaining the mixture of step (iv) at a temperature of 20 to 25 °C for 8 to 16 hours, preferably 12 hours;
(vi) isolating the product, preferably by filtration,
(vii) optionally washing the product of step (vi) with a solvent, preferably with the same solvent used in step (iv), in particular with ethyl acetate; and
(viii) drying the product of step (vii).

Drying is preferably performed under vacuum. It is also preferred to dry the product at a temperature within a range of from 35 to 65 °C, more preferably 45 to 55 °C and most preferably at about 50°C. During drying the product can be sieved to deagglomerate and/or remove lumps.

Ivabradine base used in the present invention can be prepared according to methods disclosed in EP534859 and can also be further recrystallized and/or purified to a purity level more than 99%, 99.5% or 99.9 %. If the ivabradine hydrobromide prepared by the present invention is not satisfactorily pure, it can be recrystallized to a purity level more than 99%, 99.5% or 99.9 %, determined by UPLC on an Atlantis T3 UPLC column 150 x 4,6 mm 3 µm particles, detection at 286 nm, flow rate 1.0 ml/min, column temperature 45°C, injection volume 5 µl, gradient elution, mobile phase: ammonium acetate (A) and acetonitrile (B). Alternatively the purity can be determined by HPLC. Ivabradine hydrobromide prepared according to present invention can be in an anhydrous or hydrated form. The hydrated form of ivabradine hydrobromide preferably has a water content of 1.0-6.0 % (w/w), more preferably 2.0-5.0 % (w/w), most preferably 2.0-4.0 % (w/w). Water content is determined by Karl Fisher method.

The average particle size of ivabradine hydrobromide prepared according to the present invention can be in the range of 0.1-600 µm, preferably 0.3 - 300 µm, most preferably 0.5 - 150 µm. Particle size is determined by laser diffraction method on Malvern Mastersizer 2000 instrument equiped with Hydro S dispersion unit, which is appropriate for measuring particle size distributions in the range of 20nm to 2000µm. Isopar L is used as dispersant. Usually, 100-800mg of substance is dispersed in 5-10 ml of 2% solution of epicurone (lecithin) in isopar L to get homogenuous sample suspension. The measurement is started after addition of sample suspension into dispersion unit. If agglomerates are present, homogenizing by sonication for appropriate time is used to brake-up agglomerates followed by recirculating at stirrer/pump speed rate about 40-70% of max. speed.

The term "average particle size" as used herein refers to the volume mean particle diameter, determined by laser light scattering using a Malvern Mastersizer instrument 2000.

If smaller particles are needed, the obtained ivabradine hydrobromide can be sieved, milled or micronized optionally together with other excipients. In the case of agglomerates, ultrasonication can be used.

The present invention also relates to pharmaceutical compositions containing ivabradine hydrobromide as active ingredient in admixture with one or more carriers or auxiliary substance(s) conventionally applied in the pharmaceutical industry.

The pharmaceutical compositions according to the invention usually contain 1 - 90 % by weight, preferably 1 - 50 % by weight, most preferably 1 - 15 % by weight of active ingredient. Further, pharmaceutical compositions contain an amount of ivabradine hydrobromide, corresponding to 1-30 mg ivabradine per dosage unit. Most preferably, pharmaceutical compositions according to the present invention contain an amount of ivabradine hydrobromide, corresponding to 5 mg and 7.5 mg of ivabradine per dosage unit.

The pharmaceutical compositions of the present invention may be suitable for oral (for example powders, pellets, tablets, coated tablets, capsules, orally disintegrating tablets, chewable tablets, solutions, suspensions or emulsions), parenteral (for example injection solutions for intravenous, intramuscular, subcutaneous or intraperitoneal use), rectal (suppositories), transdermal (patches) or local (ointments or patches) administration or for the application in form of implants. A tablet for oral administration is preferred pharmaceutical dosage form.

The solid pharmaceutical compositions containing ivabradine hydrobromide further comprise at least one more auxiliary agent selected from the group of fillers or carriers, binding agents, disintegrants, stabilizers, lubricants, glidants, surface active agents, sweetening agents, flavours etc.

Diluents may be selected (but are not limited to) from the group of lactose in different forms (anhydrous, monohydrate, spray dried lactose etc.), microcrystalline cellulose (such as commercially available Avicel PH 101, Avicel PH 102 or Avicel PH 112), powdered cellulose, Cellactose (mixture of lactose and cellulose powder), silicified microcrystalline cellulose, calcium phosphate, calcium hydrogen phosphate, calcium carbonate, sucrose, glucose, fructose, dextrates, maltodextrins, other sugars such as mannitol, lactitol, xylitol, sorbitol, calcium lactate or combined diluents. Starches such as pregelatinized starch, can also be used as a diluent.

Suitable binder may be selected from the group consisting of microcrystalline cellulose, hydroxypropylcellulose, low-substituted hydroxypropylcellulose, hydroxyethylcellulose, hydroxypropylmethylcellulose or other cellulose ethers, dextrates, maltodextrin, povidone (polyvinylpyrrolidone), polyethylene glycol, starch, pregelatinized starch, gelatine, polymethacrylate.

Further, disintegrants and/or superdisintegrants may also be included into pharmaceutical composition, such as starches (e.g. maize starch, potato starch), modified starches (sodium starch glycolate), modified cellulose (croscarmellose, i.e. cross-linked carboxymethlycellulose sodium), cross-linked polyvinylpyrrolidone (crospovidone), microcrystalline cellulose, carboxymethylcellulose sodium, Amberlite^{®} (polacrilin potassium), alginic acid, sodium alginate, guar gum, gellan gum, xantan gum or calcium silicate.

In addition, pharmaceutical composition may contain stabilizers, such as cellulose derivatives, polyvinylpyrrolidone and derivatives thereof, xantan gum, pectins, alginates, tragacanth, arabic gum, carrageenans, agar, bentonite, cyclodextrins, sodium hydrogen carbonate, calcium carbonate, calcium chloride, magnesium oxide, magnesium hydroxide, magnesium aluminium silicate, sodium hydrogen phosphate, sodium dihydrogen phosphate, potassium acetate, sodium tartrate or any other stabilizer.

Further, the pharmaceutical composition of the present invention may contain lubricants. Suitable lubricants are stearic acid, magnesium stearate, calcium stearate, sodium laurylsulphate, hydrogenated vegetable oil, hydrogenated castor oil, sodium stearyl fumarate, talc, macrogols. A preferred lubricant is magnesium stearate or sodium stearyl fumarate.

Glidants can also be added to the composition according to the invention. They can be selected from the group comprising of talc, silicon dioxide of different grades (such as colloidal or precipitated silica) etc.

Excipients may have multiple functions, i.e. one excipient may be diluent and additionally binder, binder and disintegrant etc.

Particularly preferred pharmaceutical compositions according to the present invention comprise:
5 to 20 wt.-%, preferably 5 to 10 wt.-% and most preferably 5 to 7 wt.-% ivabradine hydrobromide;
20 to 95 wt.-%, preferably 60 to 93 wt.-% and most preferably 60 to 90 wt.-% diluent;
0 to 40 wt.-%, preferably 0 to 30 wt.-% and most preferably 2 to 26 wt.-% binder;
0 to 40 wt.-%, preferably 0 to 30 wt.-% and most preferably 2 to 20 wt.-% disintegrant;
0 to 3 wt.-%, preferably 0.5 to 2.0 wt.-% and most preferably 0.5 to 1.0 wt.-% lubricant; and
0 to 2 wt.-%, preferably 0.1 to 1.0 wt.-% and most preferably 0.1 to 0.5 wt.-% glidant.

All percentages are based on the total mass of the composition without any optional coating.

According to a still further aspect of the present invention there is provided a process for the preparation of the pharmaceutical compositions containing hydrobromide, which comprises admixing said active ingredient with at least one carrier(s) or auxiliary substance(s) conventionally applied in the pharmaceutical industry. Any suitable process for the preparation of the tablets for oral administration may be applied, e.g. direct compression, wet (water or alcohol) or dry granulation etc.

Optionally, cores/tablets can be coated with conventional materials used for film coating, i.e. as described in Pharmaceutical Coating Technology (G. Cole (ed.), 1995). Film coating formulations usually contain the following components:
- polymer(s),
- plasticizer(s),
- colourant(s)/opacifier(s),
- vehicle(s).

The polymers used in film coating are either cellulose derivatives, such as the cellulose ethers (e.g. hydroxyethylcellulose, hydroxypropylcellulose, hydroxypropylmethylcellulose and methylcellulose), or acrylic polymers and copolymers. Occasionally, high molecular weight polyethylene glycols, polyvinyl pyrrolidone, polyvinyl alcohol and waxy materials are used. Usually their function is to prevent bad mouth feel and/or taste and in some cases degradation, e.g. oxidation of the active ingredients and/or excipients used.

The commonly used plasticizers are polyols (glycerol, propylene glycol, polyethylene glycols), organic esters (phthalate esters, dibutyl sebacetate, citrate esters, triacetin) and oils/glycerides (castor oil, acetylated monoglycerides, fractionated coconut oil).

Colourants/opacifiers are classified into several groups: organic dyes and their lakes, inorganic colours, natural colours.

Different materials from each group can also be combined in defined ratios. Film coating dispersions can also be used as ready-to-make preparations which are available on the market.

Film coating dispersion can be prepared by using different solvents (water, alcohols, ketones, esters, chlorinated hydrocarbons), preferably water.

A composition of coating dispersion (calculated on dry material) which comprises:
- 1-99%, preferably 1-95% by weight of polymer,
- 1-50%, preferably 1-40% by weight of plasticizer,
- 0.1-20%, preferably 0.1-10% by weight of colourant/opacifier,
is particularly preferred.

Conventional equipment for applying a coating, such as coating pans or Wurster coating system can be used. The mass ratio of coating to core is preferably from 0.01:1 to 0.05:1, most preferably from 0.02:1 to 0.04:1.

The pharmaceutical composition of ivabradine hydrobromide might be processed and packed under a modified atmosphere. The atmosphere with the modified oxygen content or reduced oxygen partial pressure may be obtained by the use of a reduced pressure atmosphere, e.g. by creating a partial vacuum by means of a suitable pump or by partial freezing or liquefying the atmosphere, or by the use of an inert gas, such as nitrogen or argon, or by the use of oxygen absorbents, even though the high efficacy of such absorbents leading to very low oxygen levels is normally not necessary.

The composition is present in a packaging, with a blister packaging or a bottle being preferred. Optionally, the packaging can be provided with means for trapping and disposal of free oxygen. The composition might be enclosed in a substantially gas exchange non-permeable material as packaging which has an atmosphere with the required reduced oxygen content. The substantially gas exchange non-permeable packaging is preferably selected from the group consisting of an Al/Al blister package, an Al-polychloro-3-fluoroethylene homopolymer/PVC laminate blister or a bottle.

The invention is illustrated by reference to the following examples. However, the examples are not intended to limit any scope of the claim anyway.

### EXAMPLES:

### I. SYNTHESIS

### Example 1:

14.5 ml (0.128 mole) 48 % aqueous solution of hydrogen bromide was added to the solution of 60.0 g (0.128 mole) ivabradine in 600 ml of methylene chloride at 20-25°C. The solvent was evaporated under vacuum to dryness. 400 ml of ethyl acetate was added to residue and maintained for 12 hours at 20-25°C. The product was filtered, washed with 50 ml of ethyl acetate and dried in a vacuum dryer at final temperature 50°C. During drying the product was sieved to deagglomerate lumps. 61.5 g of crystalline ivabradine hydrobromide hydrate was obtained (assay of water 3.6 %).

### Example 2:

1.15 ml (0.01 mole) 48 % aqueous solution of hydrogen bromide was added to the solution of 4.7 g (0.01 mole) ivabradine in 50 ml of ethyl acetate at 20-25°C. The suspension was heated to reflux (76°C), stirred for 10 minutes and gradually cooled to 20-25°C. 400 ml of ethyl acetate was added to residue and maintained for 12 hours at 20-25°C. The product was filtered, washed with 4 ml of ethyl acetate and dried in a vacuum dryer at final temperature 50°C. 3.2 g of crystalline ivabradine hydrobromide hydrate was obtained (assay of water 3.7 %).

### Example 3:

1.5 g of ivabradine hydrobromide as amorphous form was suspended in 10 ml of acetonitrile, heated to temperature of reflux, mixed for 10 minutes and gradually cooled to 20-25°C. The product was filtered, washed with acetonitrile and dried in a vacuum dryer at final temperature 40°C. 1.1 g of crystalline ivabradine hydrobromide hydrate was obtained (assay of water 3.7%).

### Example 4:

2.3 ml (0.02 mole) 48 % aqueous solution of hydrogen bromide was added to the solution of 9.4 g (0.02 mole) ivabradine in 100 ml of methylene chloride at 20-25°C. The solvent was evaporated under vacuum to dryness to obtain 10.9 g amorphous product (assay of water 8.4 %).

### II. PHARMACEUTICAL FORMULATIONS

### A.) Direct compression

### Example 5: 7.5 mg tablets

| | Amount [mg] | Amount by weight [%] |
|---|---|---|
| Ivabradine hydrobromide | 8.796* | 5.864 |
| Lactose monohydrate | 103.704 | 69.136 |
| Microcrystalline cellulose | 35.250 | 23.500 |
| Magnesium stearate | 1.500 | 1.000 |
| Colloidal silicon dioxide | 0.750 | 0.500 |
| Total | 150 | 100 |

| | | |
|---|---|---|
| *corresponding to 7.5 mg of ivabradine | | |

Ivabradine hydrobromide, lactose monohydrate and microcrystalline cellulose were homogeneously mixed in a suitable mixer. Optionally, suitable stabilizer may be included. Colloidal silicon dioxide and magnesium stearate were added in the end and lubricated compression mixture was pressed into tablets. Optionally, tablets are coated with the coating dispersion.

### Example 6: 7.5 mg tablets

| | Amount [mg] | Amount by weight [%] |
|---|---|---|
| Ivabradine hydrobromide | 8.796* | 5.864 |
| Maize starch | 7.500 | 5.000 |
| Cellactose | 117.204 | 78.136 |
| Pregelatinized starch | 15.000 | 10.000 |
| Magnesium stearate | 1.500 | 1.000 |
| Total | 150 | 100 |

| | | |
|---|---|---|
| *corresponding to 7.5 mg of ivabradine | | |

Ivabradine hydrobromide, maize starch, Cellactose and pregelatinized starch were homogeneously mixed in a suitable mixer. Optionally, suitable stabilizer may be included. Magnesium stearate was added in the end and lubricated compression mixture was pressed into tablets. Optionally, tablets are coated with the coating dispersion.

### Example 7: 7.5 mg tablets

| | Amount [mg] | Amount by weight [%] |
|---|---|---|
| Ivabradine hydrobromide | 8.796* | 5.864 |
| Lactose monohydrate | 99.954 | 66.636 |
| Microcrystalline cellulose | 27.000 | 18.000 |
| PVP K30 | 9.000 | 6.000 |
| Crospovidone | 3.000 | 2.000 |
| Magnesium stearate | 1.500 | 1.00 |
| Colloidal silicon dioxide | 0.750 | 0.50 |
| Total | 150 | 100 |

| | | |
|---|---|---|
| *corresponding to 7.5 mg of ivabradine | | |

Ivabradine hydrobromide, lactose monohydrate, microcrystalline cellulose, PVP K30, crospovidone and colloidal silicon dioxide were homogeneously mixed in a suitable mixer. Magnesium stearate was added in the end and lubricated compression mixture was pressed into tablets. Optionally, tablets are coated with the coating dispersion

### Example 8: 7.5 mg tablets

| | Amount [mg] | Amount by weight [%] |
|---|---|---|
| Ivabradine hydrobromide | 8.796* | 5.864 |
| Lactose monohydrate | 99.954 | 66.636 |
| Microcrystalline cellulose | 30.000 | 20.000 |
| Povidone (PVP K30) | 9.000 | 6.000 |
| Magnesium stearate | 1.500 | 1.000 |
| Colloidal silicon dioxide | 0.750 | 0.500 |
| Total | 150 | 100 |

| | | |
|---|---|---|
| *corresponding to 7.5 mg of ivabradine | | |

Ivabradine hydrobromide, lactose monohydrate, microcrystalline cellulose, PVP K30 and colloidal silicon dioxide were homogeneously mixed in a suitable mixer. Magnesium stearate was added in the end and lubricated compression mixture was pressed into tablets. Tablets were film-coated, either with the coating composition presented in the following table or with coating system Opadry^{®}.

| **Film-coating** | Amount/tablet [mg] |
|---|---|
| Hypromellose 6cp | 3.000 |
| Polyethylene glycol 6000 | 0.480 |
| Glycerol | 0.195 |
| Titanium dioxide | 0.675 |
| Iron oxide, yellow | 0.090 |
| Iron oxide, red | 0.030 |
| Magnesium stearate | 0.030 |
| Total | 4.5 |

### Example 9: 7.5 mg tablets

| | Amount [mg] | Amount by weight [%] |
|---|---|---|
| Ivabradine hydrobromide | 8.796* | 5.864 |
| Lactose monohydrate | 96.954 | 64.636 |
| Microcrystalline cellulose | 30.000 | 20.000 |
| Povidone (PVP K30) | 9.000 | 6.000 |
| Croscarmellose sodium | 3.000 | 2.000 |
| Magnesium stearate | 1.500 | 1.000 |
| Colloidal silicon dioxide | 0.750 | 0.500 |
| Total | 150 | 100 |

| | | |
|---|---|---|
| *corresponding to 7.5 mg of ivabradine | | |

Ivabradine hydrobromide, lactose monohydrate, microcrystalline cellulose, PVP K30, croscarmellose sodium and colloidal silicon dioxide were homogeneously mixed in a suitable mixer. Magnesium stearate was added in the end and lubricated compression mixture was pressed into tablets. Optionally, tablets are film coated with the aqueous coating dispersion in a suitable coating pan.

### Example 10: 7.5 mg tablets

| | Amount [mg] | Amount by weight [%] |
|---|---|---|
| Ivabradine hydrobromide | 8.796* | 5.864 |
| Lactose monohydrate | 96.954 | 64.636 |
| Microcrystalline cellulose | 30.000 | 20.000 |
| L-HPC type LH 11** | 9.000 | 6.000 |
| Croscarmellose sodium | 3.000 | 2.000 |
| Magnesium stearate | 1.500 | 1.000 |
| Colloidal silicon dioxide | 0.750 | 0.500 |
| Total | 150 | 100 |

| | | |
|---|---|---|
| * corresponding to 7.5 mg of ivabradine ** low substituted hydroxypropyl cellulose | | |

Ivabradine hydrobromide, lactose monohydrate, microcrystalline cellulose, L-HPC LH 11, croscarmellose sodium and colloidal silicon dioxide were homogeneously mixed in a suitable mixer. Magnesium stearate was added in the end and lubricated compression mixture was pressed into tablets. Optionally, tablets are film coated with the aqueous coating dispersion in a suitable coating pan.

### B.) Wet granulation

### Example 11: 7.5 mg tablets

| | Amount [mg] | Amount by weight [%] |
|---|---|---|
| Ivabradine hydrobromide | 8.796* | 5.864 |
| Maize starch | 27.000 | 18.000 |
| Lactose monohydrate | 104.454 | 69.636 |
| Maltodextrin | 7.500 | 5.000 |
| Magnesium stearate | 1.500 | 1.000 |
| Colloidal silicon dioxide | 0.750 | 0.500 |
| Granulating liquid | q.s. | Evaporates during the process |
| Total | 150 | 100 |

| | | |
|---|---|---|
| * corresponding to 7.5 mg of ivabradine "q.s." means "as needed" | | |

Ivabradine hydrobromide, maize starch, lactose monohydrate and maltodextrin were homogeneously mixed in a suitable mixer. Optionally, suitable stabilizer may be included. The obtained mixture was granulated with granulating liquid (purified water, alcohol). The wet granulate was dried, sieved and mixed with colloidal silicon dioxide and magnesium stearate. Lubricated compression mixture was pressed into tablets. Optionally, tablets are coated with the coating dispersion in a suitable coating pan.

### Example 12: 7.5 mg tablets

| | Amount [mg] | Amount by weight [%] |
|---|---|---|
| Ivabradine hydrobromide | 8.796* | 5.864 |
| Lactose monohydrate | 104.454 | 69.636 |
| Microcrystalline cellulose | 30.000 | 20.000 |
| Polyvinylpyrrolidone | 4.500 | 3.000 |
| Magnesium stearate | 1.500 | 1.000 |
| Colloidal silicon dioxide | 0.750 | 0.500 |
| Granulating liquid | q.s. | Evaporates during the process |
| Total | 150 | 100 |

| | | |
|---|---|---|
| * corresponding to 7.5 mg of ivabradine "q.s." means "as needed" | | |

The mixture of ivabradine hydrobromide, lactose monohydrate, microcrystalline cellulose, polyvinylpyrrolidone and optionally stabilizer was wetted with granulating liquid (purified water, alcohol) and kneaded. The wet granules were dried and sieved. In the end colloidal silicon dioxide and magnesium stearate were added to the dry granulate and the obtained compression mixture was compressed into tablets. Optionally, tablets are coated with the coating dispersion in a suitable coating pan.

### Example 13: 7.5 mg tablets

| | Amount [mg] | Amount by weight [%] |
|---|---|---|
| Ivabradine hydrobromide | 8.796* | 5.864 |
| Lactose monohydrate | 97.254 | 64.836 |
| Microcrystalline cellulose | 33.750 | 22.500 |
| Hydroxypropylcellulose | 9.000 | 6.000 |
| Magnesium stearate | 0.750 | 0.500 |
| Colloidal silicon dioxide | 0.450 | 0.300 |
| Granulating liquid | q.s. | Evaporates during the process |
| Total | 150 | 100 |

| | | |
|---|---|---|
| * corresponding to 7.5 mg of ivabradine "q.s." means "as needed" | | |

The mixture of ivabradine hydrobromide, lactose monohydrate, hydroxypropylcellulose and optionally stabilizer was wetted with granulating liquid (purified water, alcohol) and kneaded. The wet granules were dried and sieved. Microcrystalline cellulose and colloidal silicon dioxide were added to the dry granulate. Magnesium stearate was admixed and the obtained compression mixture was compressed into tablets. Optionally, tablets are coated with the coating dispersion in a suitable coating pan.

### C.) Dry granulation

### Example 14: 7.5 mg tablets

| | Amount [mg] | Amount by weight [%] |
|---|---|---|
| Ivabradine hydrobromide | 8.796* | 5.864 |
| Lactose monohydrate | 104.454 | 69.636 |
| Microcrystalline cellulose | 30.000 | 20.000 |
| Crospovidone | 6.000 | 4.000 |
| Magnesium stearate | 0.750 | 0.500 |
| Total | 150 | 100 |

| | | |
|---|---|---|
| * corresponding to 7.5 mg of ivabradine | | |

Ivabradine hydrobromide was mixed in a suitable mixer with all other excipients (except a part of crospovidone and a part of magnesium stearate). Optionally, suitable stabilizer may be included. The mixture was compacted with roller compactor, the obtained compacts were milled and sieved, blended with the remaining crospovidone and magnesium stearate and compressed into tablets. Optionally, tablets are coated with the coating dispersion in a suitable coating pan.

### Example 15: 7.5 mg tablets

| | Amount [mg] | Amount by weight [%] |
|---|---|---|
| Ivabradine hydrobromide | 8.796* | 5.864 |
| Lactose anhydrous | 103.704 | 69.136 |
| Microcrystalline cellulose | 30.000 | 20.000 |
| Hydroxypropylcellulose | 6.000 | 4.000 |
| Magnesium stearate | 1.500 | 1.000 |
| Total | 150 | 100 |

| | | |
|---|---|---|
| * corresponding to 7.5 mg of ivabradine | | |

Ivabradine hydrobromide was mixed in a suitable mixer with all other excipients except magnesium stearate. Optionally, suitable stabilizer may be included. The mixture was compacted with roller compactor, the obtained compacts were milled and sieved, blended with magnesium stearate and compressed into tablets. Optionally, tablets are coated with the coating dispersion in a suitable coating pan.

## Claims

1. Crystalline ivabradine hydrobromide, **characterized by** an X-ray powder diffraction pattern having peaks at 13.2, 15.8, 16.8, 21.1, 24.5, 25.7 ± 0.2 degrees two-theta, determined with CuKα radiation, 0.154178 nm.

2. Ivabradine hydrobromide according to claim 1, which is further **characterized by** having peaks at 13.2, 15.8, 16.8, 21.1, 24.5, 25.7, 26.3, 27.7 ± 0.2 degrees two-theta.

3. Ivabradine hydrobromide according to any one of the previous claims having an average particle size of 0.1 to 600 µm, preferably 0.3 to 300 µm.

4. Ivabradine hydrobromide according to any one of the previous claims comprising 1.0 to 6.0 % (w/w) of water.

5. Pharmaceutical composition comprising ivabradine hydrobromide according to any previous claim and pharmaceutically acceptable excipients.

6. The pharmaceutical composition of claim 5 comprising 1 - 90 % by weight, preferably 1 - 50 % by weight, most preferably 1 - 15 % by weight of ivabradine hydrobromide.

7. The pharmaceutical compositions of claim 6 comprising
5 to 20 wt.-%, preferably 5 to 10 wt.-% and most preferably 5 to 7 wt.-% ivabradine hydrobromide;
20 to 95 wt.-%, preferably 60 to 93 wt.-% and most preferably 60 to 90 wt.-% diluent;
0 to 40 wt.-%, preferably 0 to 30 wt.-% and most preferably 2 to 26 wt.-% binder;
0 to 40 wt.-%, preferably 0 to 30 wt.-% and most preferably 2 to 20 wt.-% disintegrant;
0 to 3 wt.-%, preferably 0.5 to 2 wt.-% and most preferably 0.5 to 1 wt.-% lubricant; and
0 to 2 wt.-%, preferably 0.1 to 1 wt.-% and most preferably 0.1 to 0.5 wt.-% glidant,
based on the total mass of the composition.

8. The pharmaceutical composition according to any one of claims 5 to 7 comprising a film coating.

9. The pharmaceutical composition of claim 8, wherein the film coating comprises:
- 1-99%, preferably 1-95% by weight of polymer,
- 1-50%, preferably 1-40% by weight of plasticizer,
- 0.1-20%, preferably 0.1-10% by weight of colourant/opacifier,
based on the total mass of the coating.

10. A process for preparing ivabradine according to any one of claims 1 to 3 comprising the steps of
(i) dissolving ivabradine in a suitable solvent, preferably in methylene chloride ;
(ii) adding a solution of hydrogen bromide, preferably an alcoholic solution or more preferably an aqueous solution, most preferably a 48 % aqueous solution of hydrogen bromide;
(iii) evaporating the solvent to dryness, preferably under vacuum,
and optionally
(iv) redissolving or dispersing the product of step (iii) in a second solvent, preferably in ethyl acetate;
(v) maintaining the mixture of step (iv) at a temperature of 20 to 25 °C for 8 to 16 hours, preferably 12 hours;
(vi) isolating the product, preferably by filtration,
(vii) optionally washing the product of step (vi) with a solvent, preferably with the same solvent used in step (iv), in particular with ethyl acetate; and
(viii) drying the product of step (vii).

## Patentansprüche

1. Kristallines Ivabrandin Hydrobromid, **gekennzeichnet durch** ein Pulver-Röntgenbeugungsdiagramm, das Signale bei 13,2, 15, 8, 16, 8, 21,1, 24,5, 25,7 ± 0, 2° 2θ aufweist, bestimmt mit CuKα-Strahlung, 0,154178 nm.

2. Ivabradin Hydrobromid gemäß Anspruch 1, das weiterhin **dadurch gekennzeichnet ist, dass** es Signale bei 13,2, 15,8, 16, 8, 21, 1, 24,5, 25,7, 26,3, 27,7 ± 0, 2° 2θ aufweist.

3. Ivabradin Hydrobromid gemäß einem der vorangehenden Ansprüche, das eine mittlere Partikelgröße von 0,1 bis 600 µm, vorzugsweise 0,3 bis 300 µm hat.

4. Ivabradin Hydrobromid gemäß einem der vorhergehenden Ansprüche, das 1,0 bis 6,0 Gew.-% Wasser enthält.

5. Pharmazeutische Zusammensetzung, die Ivabradin Hydrobromid gemäß einem der vorangehenden Ansprüche und pharmazeutisch verträgliche Hilfsstoffe enthält.

6. Pharmazeutische Zusammensetzung nach Anspruch 5, die 1 bis 90 Gew.-%, vorzugsweise 1 bis 50 Gew.-%, besonders bevorzugt 1 bis 15 Gew.-% Ivabradin Hydrobromid enthält.

7. Pharmazeutische Zusammensetzung nach Anspruch 6, die
5 bis 20 Gew.-%, vorzugsweise 5 bis 10 Gew.-% und am meisten bevorzugt 5 bis 7 Gew.-% Ivabradin Hydrobromid;
20 bis 95 Gew.-%, vorzugsweise 60 bis 93 Gew.-% und am meisten bevorzugt 60 bis 90 Gew.-% Verdünnungsmittel;
0 bis 40 Gew.-%, vorzugsweise 0 bis 30 Gew.-% und am meisten bevorzugt 2 bis 26 Gew.-% Bindemittel;
0 bis 40 Gew.-%, vorzugsweise 0 bis 30 Ges.-% und am meisten bevorzugt 0 bis 20 Gew.-% Sprengmittel;
0 bis 3 Gew.-%, vorzugsweise 0,5 bis 2 Ges.-% und am meisten bevorzugt 0,5 bis 1 Gew.-% Schmiermittel; und
0 bis 2 Ges.-%, vorzugsweise 0,1 bis 1 Gew.-% und am meisten bevorzugt 0,1 bis 0,5 Gew.-% Gleitmittel
enthält, basierend auf der Gesamtmasse der Zusammensetzung.

8. Pharmazeutische Zusammensetzung nach einem der Ansprüche 5 bis 7, die eine Beschichtung aufweist.

9. Pharmazeutische Zusammensetzung nach Anspruch 8, bei der die Beschichtung
- 1 bis 99%, vorzugsweise 1 bis 95 Gew.-% Polymer,
- 1 bis 50%, vorzugsweise 1 bis 40 Gew.-% Weichmacher,
- 0,1 bis 20%, vorzugsweise 0,1 bis 10 Gew.-% Farbstoff/Trübungsmittel enthält,
basierend auf der Gesamtmasse der Beschichtung.

10. Verfahren zur Herstellung von Ivabradin gemäß einem der Ansprüche 1 bis 3, das die folgenden Schritte aufweist:
(i) Lösen von Ivabradin in einem geeigneten Lösungsmittel, vorzugsweise in Methylenchlorid;
(ii) Zugabe einer Lösung von Bromwasserstoff, vorzugsweise einer alkoholischen Lösung oder besonders bevorzugt einer wässrigen Lösung, am meisten bevorzugt einer 48%-igen wässrigen Lösung von Bromwasserstoff;
(iii) Abdampfen des Lösungsmittels zur Trockene, vorzugsweise unter Vakuum;
und optional
(iv) Wiederauflösen oder Dispergieren des Produkts auch Schritt (iii) in einem zweiten Lösungsmittel, vorzugsweise in Ethylacetat;
(v) Halten der Mischung aus Schritt (iv) bei einer Temperatur von 20 bis 25°C für 8 bis 16 Stunden, vorzugsweise 12 Stunden;
(vi) Isolieren des Produkts, vorzugsweise durch Filtration,
(vii) optional Waschen des Produkts aus Schritt (vi) mit einem Lösungsmittel, vorzugsweise mit demselben Lösungsmittel, das in Schritt (iv) verwendet wurde, insbesondere mit Ethylacetat; und
(viii) Trocknen des Produkts aus Schritt (vii).

## Revendications

1. Bromhydrate d'ivabradine sous forme cristalline, **caractérisé en ce que** son diagramme de diffraction des rayons X à l'état de poudre, déterminé avec la raie Kα du cuivre de longueur d'onde 0,154178 nm, comporte des pics pour les valeurs de 13,2°, 15,8°, 16,8°, 21,1°, 24,5° et 25,7°, à ± 0,2° près, de l'angle 2θ.

2. Bromhydrate d'ivabradine conforme à la revendication 1, **caractérisé en outre en ce qu'**il donne des pics pour les valeurs de 13,2°, 15,8°, 16,8°, 21,1°, 24,5°, 25,7°, 26,3° et 27,7°, à ± 0,2° près, de l'angle 2θ.

3. Bromhydrate d'ivabradine conforme à l'une des revendications précédentes, présentant une taille moyenne de particules de 0,1 à 600 µm, et de préférence de 0,3 à 300 µm.

4. Bromhydrate d'ivabradine conforme à l'une des revendications précédentes, comprenant de 1,0 à 6,0 % en poids d'eau.

5. Composition pharmaceutique comprenant du bromhydrate d'ivabradine, conforme à l'une des revendications précédentes, et des excipients pharmacologiquement admissibles.

6. Composition pharmaceutique conforme à la revendication 5, comprenant de 1 à 90 % en poids, de préférence de 1 à 50 % en poids, et mieux encore de 1 à 15 % en poids, de bromhydrate d'ivabradine.

7. Composition pharmaceutique conforme à la revendication 6, comprenant :
- de 5 à 20 % en poids, de préférence de 5 à 10 % en poids, et mieux encore de 5 à 7 % en poids, de bromhydrate d'ivabradine,
- de 20 à 95 % en poids, de préférence de 60 à 93 % en poids, et mieux encore de 60 à 90 % en poids, d'un diluant,
- de 0 à 40 % en poids, de préférence de 0 à 30 % en poids, et mieux encore de 2 à 26 % en poids, d'un liant,
- de 0 à 40 % en poids, de préférence de 0 à 30 % en poids, et mieux encore de 2 à 20 % en poids, d'un délitant,
- de 0 à 3 % en poids, de préférence de 0,5 à 2 % en poids, et mieux encore de 0,5 à 1 % en poids, d'un lubrifiant,
- et de 0 à 2 % en poids, de préférence de 0,1 à 1 % en poids, et mieux encore de 0,1 à 0,5 % en poids, d'un agent de glissement,
par rapport au poids total de la composition.

8. Composition pharmaceutique conforme à l'une des revendications 5 à 7, comprenant un enrobage pelliculaire.

9. Composition pharmaceutique conforme à la revendication 8, dans laquelle l'enrobage pelliculaire comprend :
- de 1 à 99 % en poids, et de préférence de 1 à 95 % en poids, d'un polymère,
- de 1 à 50 % en poids, et de préférence de 1 à 40 % en poids, d'un plastifiant,
- et de 0,1 à 20 % en poids, et de préférence de 0,1 à 10 % en poids, d'un agent colorant et/ou opacifiant,
par rapport au poids total de l'enrobage.

10. Procédé de préparation de bromhydrate d'ivabradine conforme à l'une des revendications 1 à 3, comportant les étapes suivantes :
i) dissoudre de l'ivabradine dans un solvant approprié, et de préférence, dans du chlorure de méthylène ;
ii) ajouter une solution de bromure d'hydrogène, de préférence une solution alcoolique ou mieux encore une solution aqueuse, le mieux étant une solution aqueuse à 48 % de bromure d'hydrogène ;
iii) chasser le solvant par évaporation à siccité, de préférence sous vide ;
et en option,
iv) redissoudre ou disperser le produit issu de l'étape (iii) dans un deuxième solvant, et de préférence, dans de l'acétate d'éthyle ;
v) maintenir le mélange issu de l'étape (iv) à une température de 20 à 25 °C durant 8 à 16 heures, et de préférence, durant 12 heures ;
vi) isoler le produit, de préférence par filtration ;
vii) en option, laver le produit issu de l'étape (vi) avec un solvant, et de préférence, avec du même solvant que celui utilisé dans l'étape (iv), en particulier de l'acétate d'éthyle ;
viii) et faire sécher le produit issu de l'étape (vii).
